# EUROPEAN PATENT SPECIFICATION

(11) **EP 1 148 041 B1**
(45) Date of publication and mention of the grant of the patent: **17.09.2008**
(21) Application number: 01108596.6
(22) Date of filing: 05.04.2001
(51) Int. Cl.: C07C 43/313, C07C 43/315, C08F 16/38, C07C 41/50

(54) **Fluorovinylethers and polymers obtainable therefrom**
Fluorvinylether und daraus hergestellte Polymere
Ethers fluorovinyliques et des polymères ainsi obtenus

(30) Priority: 21.04.2000 IT MI000902
(43) Date of publication of application: 24.10.2001
(73) Proprietor: Solvay Solexis S.p.A., 20121 Milano (IT)
(72) Inventor: Navarrini, Walter, Boffalora Ticino, Milano (IT)
(74) Representative: Sama, Daniele

(56) References cited:
- EP-A- 0 683 181
- EP-A- 0 976 706
- WO-A-01/46107
- WO-A-99/48939

## Description

The present invention relates to fluorovinylethers, the process for preparing them and the polymers obtainable therefrom.

It is well known that perfluoroalkylvinylethers are used as monomers for the olefin copolymerization, specifically tetrafluoroethylene, vinylidene fluoride, chlorotrifluoroethylene (CTFE), hexafluoropropene. The introduction of small amounts of perfluoroalkylvinylethers in plastomeric polymers implies a higher polymer processability and better hot mechanical properties. The introduction of high amounts of perfluorovinylethers in crosslinkable fluoropolymers implies elastomeric oroperties at low temperature of fluorinated rubbers.

The need was felt, in the fluorinated polymeric material field, to produce both plastomers having good properties at high temperatures, and elastomers having improved properties at low temperatures by using only one fluorovinylether.

Such properties at low temperatures can generally be expressed by the glass transition temperature Tg.

Furthermore the need was felt to have available amorphous or crystalline (co)polymers having a low content of COF end groups. A lower content of COF end groups leads to obtain polymers having a higher thermal stability. A lower Tg allows to have polymers which can be used at lower temperatures and therefore to have available elastomers with a wider use range. To obtain the combination of the above mentioned properties, fluorovinylethers must have a high unitary capability to modify the backbone properties, as well as high reactivity to be used as comonomers both in plastomeric and in elastomeric fluoropolymers. It was desirable to have available vinylethers obtainable by simple processes having a limited number of steps. Preferably it would be desirable to have available a continuous process for preparing said vinylethers.

To solve the above technical problem, fluorovinylethers having different structural properties, have been proposed in the prior art. However from the prior art, hereinafter described, various unsolved problems exist in the perfluorovinylether synthesis and in the preparation of the correspondig polymers having the combination of the above mentioned properties.

USP 3,132,123 describes the preparation of perfluoroalkylvinylethers, of the corresponding homopolymers and copolymers with TFE. Homopolymers are obtained under extreme experimental conditions, by using polymerization pressures 1 from 4,000 to 18,000 atm. The perfluoromethylvinylether (PMVE) homopolymer is an elastomer: the Tg is not reported. The general formula of the described vinylethers is the following:

CF₂=CFOR^{o}_{F}

wherein R^{o}_{F} is a perfluoroalkyl radical preferably from 1 to 5 carbon atoms. A process for preparing these vinylethers is described in USP 3,291,843 wherein the starting acylfluoride is salified and pyrolized with carbonates also in the presence of solvents. By this process undesired hydrogenated by-products are obtained.

USP 3,450,684 relates to vinylethers having the formula:

CF₂=CFO(CF₂CFX⁰O)ₙ,CF₂CF₂X⁰

wherein X^{o} = F, Cl, CF₃, H and n' can range from 1 to 20. Also homopolymers obtained by UV polymerization are reported. The exemplified copolymers are not characterized by their properties at low temperatures.

USP 3,635,926 relates to the emulsion copolymerization of perfluorovinylethers with TFE, showing that the presence of -COF acylfluoride end groups makes the polymers unstable. The same phenomenon was already reported in USP 3,085,083 in the perfluorovinylether polymerization systems in solvent.

USP 3,817,960 relates to the preparation and polymerization of perfluorovinylethers having the formula

CF₃O(CF₂O)_{n"}CF₂CF₂OCF=CF₂

wherein n" can range from 1 to 5. The compound synthesis is complex, it requires three steps. The preparation of the starting compound CF₃O (CF₂O) _{n"}CF₂C(O) F is carried out by oxidation at low temperature in the presence of U.V. radiations; besides the condensation with HFPO (hexafluoropropenoxide) and the subsequent alkaline pyrolisis is necessary. No data on the above indicated properties are reported. With regard to this see USP 5,910,552.

USP 3,896,179 relates to the separation of "primary" isomers of perfluorovinylethers, for example of CF₃CF₂CF₂OCF=CF₂ from the corresponding less stable "secondary" isomers CF₃-(CF₃)CFOCF=CF₂. The latter are undesired products as regards both the polymer preparation and the poor properties of the obtained polymers.

USP 4,340,750 relates to the preparation of perfluorovinylethers having the formula

CF₂=CFOCF₂R⁰_{f}X¹

wherein R⁰_{f} is a C₁-C₂₀ perfluoroalkyl optionally containing oxygen, X¹=H, Cl, Br, F, COOR⁰, CONR⁰R' wherein R⁰ is a C₁-C₁₀ alkyl group and R' represents H or a C₁-C₁₀ alkyl group. In the preparation of these compounds an acylfluoride together with iodine and tetrafluoroethylene is used, avoiding the final step of the acylfluoride pyrolisis which comes from the perfluoro-propene epoxide, by a deiodofluorination reaction, which takes place with low yields.

USP 4,487,903 relates to the preparation of fluoroelastomeric copolymers using perfluorovinylethers having the formula:

CF₂=CF(OCF₂CFY⁰)ₙ⁰OX²

wherein n° ranges from 1 to 4; Y⁰=_{F}, Cl, CF₃, H; X² can be C₁-C₃ perfluoroalkyl group, C₁-C₃ ω-hydroperfluoroalkyl group, C₁-C₃ ω-chloroperfluoroalkyl group. The polymer has a content of fluorovinylether units ranging from 15 to 50% by moles. These vinylethers give copolymers which at low temperatures have better properties than those of the above mentioned perfluorovinylethers PVE (perfluoropropylvinylether) and MVE type. In the patent it is disclosed that in order to have good properties at low temperature, the presence of at least two ether bonds in the side chain adjacent to the double bond is required. Furthermore from the patent it results that for n° values higher than 4 it is difficult to purify the monomers and the effect on the decrease of the polymer T_{g} is lower. Besides the reactivity of the described vinylethers is very low and it is difficult to obtain polymers having a high molecular weight able to give good elastomeric properties. A TFE/perfluorovinylether copolymer (n⁰=₂) 73/27 % by moles with Tg of -32°C is exemplified. However the polymer is obtained 6with very long reaction times (96 hours of polymerization).

EP 130,052 describes the perfluorovinylpolyether (PVPE) polymerization which leads to amorphous perfluoropolymers with a T_{g} ranging from -15 to -100°C. The described polymers have T_{g} values reaching up to -76°C; the further Tg decrease is obtained by using perfluoropolyethers as plasticizers. In the patent copolymers and terpolymers of TFE and MVE with vinylethers (PVPE) having the formula

CF₂=CFO(CF₂CF(CF₃)O)_{n"}R⁰_{f},

are described, wherein n"' ranges from 3 to 30 and R⁰_{f}, is a perfluoroalkyl group. Due to purification difficulties, the used vinylethers are vinylether mixtures with different n"' values. According to said patent the most evident effect on the T_{g} decrease is shown when n"' is equal to or higher than 3, preferably higher than 4. According to the polymerization examples described in said patent the final mass of the polymer, besides the hot and under vacuum treatment, must then be washed with freon^{®} TF in order to remove all the unreacted monomer (PVPE). From the Examples it results that the reactivity of all the described monomers (PVPE) is poor.

USP 4,515,989 relates to the preparation of new intermediates for the fluorovinylether synthesis. According to the patent the vinylether synthesis is improved by using an intermediate able to more easily decarboxylate. For its preparation fluoroepoxides of formula: 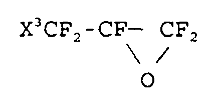 wherein X³ = Cl, Br are used.

USP 4,619,983 describes the copolymerization of VDF with vinylethers having the formula:

CF₂=CFOX⁴

wherein X⁴ is a C₃-C₉ oxyperfluoroalkyl radical containing from 1 to 3 oxygen atoms. The obtained polymers are not perfluorinated polymers and show a poor stability to alcohols.

USP 4,765,190 relates to the polymerization of perfluorovinylpolyethers (PVPE) similar to those of USP 4,487,903 with TFE and low perfluoropropene percentages, in order to increase the mechanical properties of the obtained polymers.

EP 338,755 relates to the preparation of perfluorinated copolymers by using direct fluorination of partially fluorinated copolymers. More reactive partially fluorinated monomers are used, the obtained polymers are fluorinated with elemental fluorine. The fluorination step requires a supplementary process unit, besides in this step elemental fluorine is used, which is a highly oxidizing gas, with the consequent precautions connected to its use. Besides in the patent it is stated that in order not to compromise the fluorination reaction and the properties of the obtained polymers, using the invention process the percentage of the comonomer in the polymer cannot exceed 50% by moles.

USP 5,268,405 reports the preparation of perfluorinated rubbers having a low Tg, by the use of high viscosity perfluoropolyethers as plasticizers of perfluorinated rubbers (TFE/MVE copolymers). However during the use perfluoropolyether bleeds take place. This is true especially for the PFPE having a low molecular weight (low viscosity): in said patent, therefore, the high viscosity PFPE use is suggested, and therefore the low viscosity PFPEs must previously be removed.

USP 5,350,497 relates to the preparation of perfluoroalkylvinylethers by fluorination with elemental fluorine of hydrofluorochloroethers and subsequent dechlorination.

USP 5,401,818 relates to the prepartion of perfluorovinylethers of formula:

R¹_{f} (OCF₂CF₂CF₂) _{m'}-OCF=CF₂

(wherein R¹_{f} is a C₁-C₃ perfluoroalkyl radical and m' is an integer ranging from 1 to 4) and of the corresponding copolymers having improved properties at low temperature. The preparation of said perfluorovinylethers is carried out by 7 steps, some of them have very low yields, and comprise also a fluorination with elemental F₂. The reactivity of said perfluorovinylethers is anyhow low.

As it is shown from the above prior art, the perfluorovinylether synthesis generally involves a multistep process with low yields (USP 3,132,123, USP 3,450,684), with additional purifications to remove undesired isomers (USP 3,896,179) and the need to control the undesired hydrogenated by-products (USP 3,291,843). Alternatively, in the synthesis substances acting as intermediates, which are suitably prepared, and which allow to eliminate said drawbacks (USP 4,340,750, USP 4,515,989), are used.

Furthermore in some cases the vinylether preparation requires the fluorination with elemental fluorine of partially fluorinated intermediates (USP 5,350,497); or, to avoid synthesis and low reactivity problems of the perfluorovinylethers, fluorination of partially fluorinated polymers (EP 338,755) is suggested.

Other problems shown in the prior art relate to the low reactivity of the perfluorovinylethers, which makes it necessary the recovery of the unreacted monomers from the reaction products (UK 1,514,700), and the stability problems for the polymers having -C(O)F end groups (USP 3,635,926). These last can be furtherly transformed by suitable reactants in order to increase the stability of the fluorinated polymer (EP 178,935).

Perfluorooxyalkylvinylethers are used to confer to the fluorinated rubbers good properties at low temperatures, and specifically to lower the copolymer glass transition temperature.

By increasing the perfluorooxyalkyl units forming the side perfluorooxyalkyl substituent, the T_{g} of the corresponding obtainable amorphous copolymers decreases, but at the same time the vinylether reactivity drastically decreases, making more evident the problems previously shown for the recovery of the unreacted monomer from the polymerization products or from the polymer itself (USP 4,487,903 - EP 130,052): In some cases, where the monomer cannot be completely removed by simple stripping under vacuum, more washings must then be carried out with fluorinated solvents for completely eliminating the unreacted vinylethers from the polymeric mass.

The perfluoromethylvinylether (MVE) is used as comonomer in plastomeric fluoropolymers and, at higher concentrations, also in elastomeric fluoropolymers. In particular in EP 633,257 and EP 633,274 MVE is polymerized with TFE in the presence of small amounts of PVE or dioxoles to obtain polymers with improved flex life.

The amorphous copolymers of TFE with perfluoromethylvinylether have a T_{g} around 0°C or slightly lower (Maskornik, M. et al. "ECD-006 Fluoroelastomer - A high performance engineering material". Soc. Plast Eng. Tech. Pao. (1974), 20, 675-7).

The T_{g} extrapolated value of the MVE homopolymer is of about -5°C (J. Macromol. Sci.-Phys., B1(4), 815-830, Dec. 1967) .

In USP 5,296,617 and 5,235,074 there is described the hypofluorite CF₂(OF)₂ reactivity towards unsaturated products, which contemporaneously leads to the formation of the dioxolane derivative and to the fluorination compound of the olefin itself. In EP 683,181 there is described the CF₂(OF)₂ reactivity towards olefins which leads to the formation of linear reaction compounds between one hypofluorite molecule and two molecules of the same olefin, for the preparation of symmetric dienes.

WO 99/48939 discloses perfluoroalkylvinyl ether or perfluorooxyalkylvinyl ether or mixture thereof to be used in the preparation of perfluoroelastomers. Homopolymer or copolymers obtained from said vinylethers are not described and no data of Tg or thermal stability of said polymers are reported.

WO 01/46107 discloses perfluorinated vinylethers of formula R_{f}-OCF₂O-CF=CF₂ and a method for preparing them comprising: a) starting from a specific hydrocarbon precursor having a 2-alkoxypropionate moiety; b) fluorinating the hydrocarbon precursor; c) converting the intermediate obtained above to its corresponding acid metal salt; d) converting the perfluorinated acid metal salt to its corresponding perfluorovinyl ether. Homopolymer or copolymers obtained from said vinylethers are not described and no data of Tg or thermal stability of said polymers are reported.

EP 976 706 describes fluorovinylethers (FVE) of formula CF₂=CH-O-R_{A} wherein R_{A} can be a fluoroalkyl group, optionally containing oxygen atoms, or a fluorooxyalkyl group, to be used in the copolymerization of TFE.

The Applicant has surprisingly and unexpectedly found that it is possible to solve the above technical problem as described hereinafter, by using special fluorovinylethers, which are furthermore easily synthetizable and obtainable by a continuous process.

An object of the present invention are fluorovinylethers of general formula:

CFX=CXOCF₂OR (I)

wherein R is a C₂-C₆ linear, branched or C₅-C₆ cyclic (per) fluoroalkyl group, or a C₂-C₆ linear, branched (per)fluoro oxyalkyl group containing from one to three oxygen atoms ; when R is a fluoroalkyl or fluorooxyalkyl group as above defined it can contain from 1 to 2 atoms, equal to or different from each other, selected from the following: H, Cl, Br, I; X = H.

The fluorovinylethers of general formula:

CFX=CXOCF₂OCF₂CF₂Y (II)

wherein Y = F, OCF₃; X as above defined, are preferred among the compounds of formula (I).

Surprisingly, the vinylethers according to the invention show the advantages reported hereinafter with respect to the known vinylethers.

The obtainable advantages can be attributed to the -OCF₂O- unit directly bound to the ethylene unsaturation.

The Tg lowering obtained with the vinylethers of the invention is connected to the presence of the (-OCF₂O- unit directly bound to the unsaturation. The Tg lowering is surprisingly so evident to be defined a primary effect.

In fact if the vinylether of the invention with two oxygen atoms is used:

CF₂=CF-O-CF₂-O-CF₂CF₃ (MOVE 1)

the Tg lowering is clearly higher with respect to the PVE

CF₂=CF-O-CF₂CF₂CF₃ (PVE)

and to the vinylether having the same formula, but with the second oxygen atom in a different position and without showing the characteristic unit (-OCF₂O-)

CF₂=CF-O-CF₂CF₂-O-CF₃ (*β*-PDE)

It is surprising to notice that with respect to MVE

CF₂=CF-O-CF₃

the β-PDE vinylether does not give any advantage as regards Tg.

On the contrary the primary effect of the (-OCF₂O-) unit results very clear with the vinylethers of the invention (MOVE).

Besides it has surprisingly been found that the (-OCF₂O-) unit bound to the ethylene unsaturation of the vinylethers of the invention increases the vinylether reactivity drastically reducing the rearrangements to COF which cause instability.

The advantages of the polymers of the invention can be summarized as follows:
- The reactivity of the new monomers allows to prepare copolymers having a high MW (molecular weight) with a very low content of carboxylic groups or derivatives thereof such as -C(O)F, -COO-. The carboxylic group content in the copolymer with TFE has resulted of about 10 times lower than that of a copolymer prepared under the same conditions but using perfluoropropylvinylether (PVE) instead of fluorovinylethers (see the Examples) . As said, the presence of a lower content of carboxylic groups, or of the corresponding derivatives (amides, esters, etc.) allows to obtain more stable polymers. The reactivity of the monomers of the invention is surprisingly high (see the homopolymerization Examples). The fluorovinylethers of the invention can be used as comonomers in plastomeric (per)fluoropolymers (containing crystalline domains). To obtain plastomeric polymers the amount of the vinylether of the invention must be such to lead to the formation of crystalline domains, generally < 10% by moles. Their use in the polymerization reactions with fluoroolefins allows to substantially and contemporaneously reduce two important disadvantages of the prior art: the recovery of the unreacted vinylether and the polymer instability due to the presence of carboxylic end groups. A further advantage of the fluorovinylethers of the invention, as hereinafter illustrated, consists in that their preparation is carried out in a continuous plant by a limited number of steps. Furthermore the used raw materials are cheap. The following ones can for example be mentioned:
   CF₂(OF)₂, CF₂=CF₂, CF₂=CFOCF₃, CHCl=CFCl, CFCl=CFCl, CF₂=CFCl, CF₂=CFH, CF₂=CH₂, CHCl=CHCl and other olefins.

The use of these reactants is specified in the synthesis process of the vinylethers of the invention.

Polymers, homopolymers, copolymers are obtainable by polymerizing, with at least another monomer, the fluorovinylethers having the following formulas (I)- (IV):

- CFX=CXOCF₂OR (I)

wherein R is a C₂-C₆ linear, branched or C₅-C₆ cyclic (per)fluoroalkyl group, or a C₂-C₆ linear, branched (per-)fluoro oxyalkyl group containing from one to three oxygen atoms; when R is a fluoroalkyl or fluorooxyalkyl group as above defined it can contain from 1 to 2 atoms, equal to or different from each other, selected from the following: H, Cl, Br, I; X = F, H.
- fluorovinylethers of general formula:

   CFX=CXOCF₂OCF₂CF₂Y (II)

   wherein Y = F, OCF₃; X as above defined.
- perfluorovinylethers of formula:

   CF₂=CFOCF₂OCF₂CF₂Y (III)

   wherein Y is as above defined.
- perfluorovinylether having the formula:

   CF₂=CFOCF₂OCF₂CF₃ (IV)
wherein the (per) fluoropolymers are plastomeric polymers since the amount of the vinylether of the invention is such to lead to the formation of the crystalline domains.

With copolymer, a polymer containing the vinylether of the invention and one or more comonomers, is meant.

With comonomers, fluorinated compounds having at least one polymerizable double bond C=C, optionally containing hydrogen and/or chlorine and/or bromine and/or iodine and/or oxygen, are preferably meant.

Optional comonomers which can be copolymerized are non fluorinated C₂-C₈ olefins, such as ethylene, propylene, isobutylene.

Among the usable comonomers the following can be mentioned:
- C₂-C₈ perfluoroolefins, such as tetrafluoroethylene (TFE), hexafluoropropene (HFP), hexafluoroisobutene;
- C₂-C₈ hydrogenated fluoroolefins, such as vinyl fluoride (VF), vinylidene fluoride (VDF), trifluoroethylene, CH₂=CH-R²_{f} perfluoroalkylethylenes, wherein R²_{f} is a C₁-C₆ perfluoroalkyl;
- C₂-C₈ chloro- and/or bromo- and/or iodo-fluoroolefins, such as chlorotrifluoroethylene (CTFE) and bromotrifluoroethylene;
- CF₂=CFOR²_{f} (per)fluoroalkylvinylethers (PAVE), wherin R²_{f} is a C₁-C₆ (per)fluoroalkyl, for example trifluoromethyl, bromodifluoromethyl or heptafluoropropyl;
- CF₂=CFOX^{a} (per) fluoro-oxyalkylvinylethers, wherein X^{a} is: a C₁-C₁₂ alkyl, or a C₁-C₁₂ oxyalkyl, or a C₁-C₁₂ (per)fluorooxyalkyl having one or more ether groups, for example perfluoro-2-propoxy-propyl.
- sulphonic monomers having the structure CF₂=CFOX^{b}SO₂F, wherein X^{b} = CF₂CF₂, CF₂CF₂CF₂, CF₂CF(CF₂X^{c}) wherein X^{c} = F, Cl, Br.

The process for preparing fluorinated polymers according to the present invention can be carried out by polymerization in organic solvent as described in USP 4,864,006 and 5,182,342. The organic solvent is selected from the group comprising chlorofluorocarbons, perfluoropolyethers, hydrofluorocarbons and hydrofluoroethers.

The process for preparing the polymers of the present invention can be carried out also by polymerization in aqueous emulsion according to well known methods in the prior art, in the presence of a radical initiator. This can be selected for example from: inorganic peroxides (for example alkaline metal or ammonium persulphates, perphosphates, perborates or percarbonates), optionally in combination with ferrous, cuprous or silver salts, or of other easily oxidizable metals; organic peroxides (for example, disuccinylperoxide, terbutylhydroperoxide, diterbutylperoxide) ; azocompounds (see USP 2, 5-15,628 and USP 2,520,338. It is also possible to use organic or inorganic redox systems, such as ammonium persulphate/sodium sulphite, hydrogen peroxide/aminoiminomethansulphinic acid.

In the reaction medium also surfactants of various type are usually present, among which the fluorinated surfactants of formula:

R³_{f} - X⁻ M⁺

are particularly preferred, wherein R³_{f} is a C₅-C₁₆ (per) fluoroalkyl chain or a (per)fluoropolyoxyalkyl chain, X⁻ is -COO⁻ or -SO₃⁻, M⁺ is selected from: H⁺, NH₄⁺, an alkaline metal ion. Among the most commonly used, ammonium perfluorooctanoate, (per)fluoropolyoxyalkylenes ended with one or more carboxylic groups, etc. can be mentioned.

During the polymerization, known iodinated chain transfer agents can be added to the reaction medium. It is also possible to use as chain transfer agents alkaline or earth-alkaline metal iodides and/or bromides, according to USP 5,173,553.

Other chain transfer agents are mentioned in USP 4,766,190.

Another object of the present invention consists in the synthesis process of the new (per)fluorovinylethers, which comprises the reaction of the hypofluorite with a fluorinated olefin of formula R₁R₂C=CR₃R₄ to give the intermediate hypofluorite F-CR₁R₂-CR₃R₄-OCF₂OF, the subsequent reaction of said compound with a second fluorinated olefin of formula R₅R₆C=CR₇R₈ to give the intermediate F-CR₁R₂-CR₃R₄-OCF₂O-CR₅R₆-CR₇R₈-F, which by dehalogenation or dehydrohalogenation leads to the new perfluorovinylethers.
The general scheme of the synthesis is the following:
a)

   CF₂(OF)₂ + R₁R₂C=CR₃R₄ → F-CR₁R₂-CR₃R₄-OCF₂OF (VI)
b)

   F-CR₁R₂-CR₃R₄-OCF₂OF + R₅R₆C²=C¹R₇R₈ -----→ F-CR₁R₂-CR₃R₄-OCF₂O-C²R₅R₆-C¹R₇R₈-F (VII)
c)

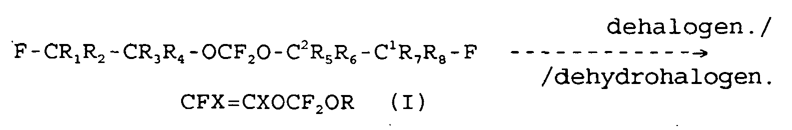

In this synthesis scheme:
- with reference to the formula of the compound (VII):
   - R₁, R₄, equal or different, are H, F; R₂, R₃, equal or different are H, Cl under the following conditions: (1) when the final reaction is a dehalogenation R₂, R₃ = Cl, (2) when the final reaction is a dehydrohalogenation one of the two substituents R₂, R₃ is H and the other is Cl;
   - R₅, R₆, R₇, R₈ are :
      - F, or one of them is a C₁-C₄ linear or branched perfluoroalkyl group, or a C₁-C₄ linear or branched perfluorooxyalkyl group containing from one to three oxygen atoms, or R₅ and R₇, or R₆ and R₈, are linked each other to form with C² and C¹ a C₅-C₆ cycle perfluoroalkyl group;
      - when one of the R₅ to R₈ radicals is a C₂-C₄ linear or branched fluoroalkyl, or a C₂-C₄ linear or branched fluorooxyalkyl group containing from one to three oxygen atoms, one or two of the other R₅ to R₉ are F and one or two of the remainders, equal to or different from each other, ar selected from H, Cl, Br, Iodine; when the substituents selected from H, Cl, Br, Iodine are two, they are both linked to the same carbon atom; when R₅ and R_{7,} or _{R}6 and R₆, are linked each other to form with C² and C¹ a C-C₆ cycle fluoroalkyl group, one of the two tree substituents R₆, R₈ or R₆, R₇ is F and the other is selected from H, Cl, Br, Iodine.
- the fluoroalkene used in the reaction a) is replaceable with that of the subsequent reaction b); in this case the meanings defined for the substituents of the R₁-R₄ group, and respectively of the R₅-R₈ group, are interchangeable each other, with the proviso that the position of each radical of each of the two groups R₁-R₄ and R₅-R₈ with respect to -OCF₂O- on the chain of the intermediate compound (VII), is the same which is occupied if the synthesis takes place according to the above reported scheme and the two olefins each react in the considered steps.

In the first reaction a) of the above illustrated scheme a hypofluorite gas flow CF₂(OF)₂, suitably diluted with an inert fluid, comes into contact, in a suitable reactor with outlet, on the bottom of the same (first reactor), with a flow of the R₁R₂C=CR₃R₄ olefin, optionally diluted in an inert fluid, to allow the chemical reaction a) with formation of the intermediate hypofluorite (VI). To favour the reaction stoichiometry, the reactants must be introduced into the reactor in an approximately unitary molar ratio, or with an excess of CF₂(OF)₂. The residence time of the mixture in the reactor can range from few hundredths of second up to about 120 seconds depending on the olefin reactivity, the reaction temperature and the presence of optional reaction solvents.

The reaction temperature can range from -40° to -150°C, preferably from -80° to -130°C.

The compound (VI) usually is not separated from the reaction product and it is transferred in a continuous way to the subsequent reaction described in step b).

The mixture of the products coming out from the first reactor can be heated at room temperature before being fed into the second reactor.

In the second reaction b) the second olefin R₅R₆C=CR₇R₈ pure or in solution, reacts with the product obtained in the first reaction with formation of compound (VII).

The olefin can be fed in a continuous way, so as to maintain its concentration constant in the reactor. The temperature of the reaction b) can range from -20° to - 130°C, preferably from -50° to -100°C. The olefin concentration is higher than or equal to 0.01M, preferably the concentration is higher than 3M, more preferably also the pure compound can be used.

The solvents used in steps a) and b) are perfluorinated or chlorohydrofluorinated solvents or hydrofluorocarbons. Examples of said solvents are: CF₂Cl₂, CFCl₃, CF₃CFH₂, CF₃CF₂CF₃, CF₃CCl₂H, CF₃CF₂Cl.
In the reaction c) the compound (VII), dependently on the olefins used in steps a) and b), after distillation from the reaction product, is subjected to dechlorination or to dehydrochlorination to obtain the vinylethers of formula (I). This last step can be carried out by using reactions widely described in the prior art. The suitable selection of the substituents R₁ to R₈ in the two olefins used in the synthesis allows to obtain the vinylethers of the present invention.

Another object of the invention is a process wherein a hypofluorite of formula X₁X₂C(OF)₂ wherein X₁ and X₂ equal or different are F, CF₃, and two fluoroalkenes of formula respectively R^{A}₁R^{A}₂C=CR^{A}₃R^{A}₄ and R^{A}₅R^{A}₆C=CR^{A}₇R^{A}₈, wherein R^{A}₁-R^{A}₈ equal or different, are F, H, Cl, Br, I, -CF₂OSO₂F, -SO₂F, -COF, C₁-C₅ linear or branched perfluoroalkyl or oxyperfluoroalkyl group, are reacted according to steps a) and b) excluding the dehalogenation or dehydrohalogenation step, to obtain compounds of general formula (VIII)

F-CR^{A}₁R^{A}₂-CR^{A}₃R^{A}₄ -OCF₂O-CR^{A}₅R^{A}₆-CR^{A}₇R^{A}₈-F.

The following Examples are reported with the purpose to illustrate the invention and they do not limit the scope of the same.

In the Examples the thermogravimetric analysis TGA is carried out by using a 10°C/min rate.

### EXAMPLE 1

### Synthesis of CF₃CF₂OCF₂OCFClCF₂Cl perfluoro-1-2, dichloro-3,5-dioxaheptane.

The used reactor is of cylindrical type, with a total volume of 300 ml and is equipped with magnetic dragging mechanical stirrer, turbine with recycle of the reacting gas placed at 20 cm from the reactor top, internal thermocouple, two internal copper pipes for the reactant feeding which end at about 1 mm from the turbine, and product outlet from the bottom. In the reactor, inside of which the temperature is maintained at -114°C, 1.1 1/h (litres/hour) of CF₂(OF)₂ and 3.3 1/h of He are introduced through one of the two inlet pipes; A flow of 1.1 1/h of CF₂=CF₂ and 0.7 1/h of He is maintained through the second inlet pipe. Feeding is continued for 6.6 hours.

The residence time of the transport gas in the reaction zone comprised between the outlet of the two feeding pipes in the reactor and the inlet of the discharge pipe is of about 4 sec.

On the reactor bottom the reaction products are brought to room temperature and the gaseous mixture flow, monitored by gaschromatography, is fed in a continuous way, under mechanical stirring, into a second reactor having a 250 ml volume maintained at the temperature of -70°C, equipped with mechanical stirrer, thermocouple, dipping inlet for the reacting mixture, outlet with head of inert gas. The reactor contains 72.6 g of dichlorodifluoroethylene CFCl=CFCl.

At the end of the addition of reacting gases into the second reactor, the reaction raw material is distilled by a plate column at atmospheric pressure, collecting 41.5 g of the desired product (boiling point 91°C).

The yield of perfluoro-1,2 dichloro-3,5-dioxaheptane, calculated with respect to CF₂(OF)₂, is of 36%.

### Characterization of prfluoro 1,2, dichloro-3,5-dioxaheptane.

Boiling point at atmospheric pressure: 91°C.
¹⁹F-NMR spectrum in p.p.m. (with respect to CFCl₃ = O):
-51.3/-53.0 (2F, O-CF₂-O) ; -70.6/-72.6 (2F, -C-CF₂Cl);
-78.0/-78.4 (1F, O-CFCl-C) ; -87.8 (3F, CF₃-C) ;
-90.2/-91.8 (2F, C-CF₂-O).

Mass spectrum (E.I. electronic impact), main peaks and respective intensities:
69 (48.6%); 119 (84.3%); 151 (76.8%); 153 (69.8%); 185 (100%).
IR spectrum (cm⁻¹) intensity : (w) =weak, (m) =medium, (s)=strong, (vs)=very strong:
1407.3 (w) ; 1235.8 (vs) ; 1177.7 (vs) ; 1097.5 (vs) ; 1032.2 (s) ; 929.3 (w) ; 847.9 (m).

### EXAMPLE 2

### Synthesis of CF₃OCF₂CF₂OCF₂OCFClCF₂Cl perfluoro-1,2-dichloro-3,5,8-trioxanonane (isomer A) and CF₃OCF(CF₃)OCF₂OCFClCF₂Cl perfluoro-1,2-dichloro-3,5,7-trioxa-6-methyloctane (isomer B).

In a reactor identical to that used in Example 1, maintained at the same temperature of -114°C, 1.55 1/h of CF₂(OF)₂ and 4.5 1/h of He are introduced through one of the two inlet pipes; through the second inlet pipe 1.4 1/h of CF₂=CF-OCF₃ and 0.7 1/h of He for 4.5 hours.

The residence time of the transport gas in the reaction zone comprised between the reactor outlet and the end of the two feeding pipes is of about 3 sec.

On the reactor bottom the reaction products are brought to room temperature and the gaseous mixture flow, monitored by gaschromatography, is fed in a continuous way, under mechanical stirring, into a second reactor identical to the one used for the same step in Example 1. Inside, where a temperature of -70°C is maintained, there are 51 g of dichlorofluoroethylene CFCl=CFCl.

At the end of the addition of the reacting gases into the second reactor, the reaction raw material is distilled by a plate column at the reduced pressure of 250 mmHg. 50 g of a mixture formed by two isomers, respectively, isomer A) perfluoro-1,2-dichloro-3,5,8-trioxanonane and isomer B) perfluoro-1,2-dichloro-3,5,7-trioxa-6-methyloctane are collected. The mixture composition is determined by gaschromatography and is the following: isomer A 79%, isomer B 21%. The molar yield of A+B with respect to the used CF₂(OF)₂ is 38%. The molar yield of A+B with respect to the used perfluoromethylvinylether is 42%. The isomers have been separated by preparative gaschromatography.

### Characterization of products A and B

Mixture boiling point (A 79%, B 21%) at the reduced pressure of 250 mmHg: 82°C.

¹⁹F-NMR spectrum in p.p.m. (with respect to CFCl₃=0) of the isomer A:

| | |
|---|---|
| -50.6/-52.4 (2F, O-CF₂-O) ; | -70.0/-71.8 (2F, C-CF₂Cl) ; |
| -77.7 (1F, O-CFCl-C) ; | -55.3/-55.6 (3F, CF₃-OC) ; |
| -90.7/-91.1 (2F, C-OCF₂-C) ; | -90.2/-90.6 (2F, C-OC-CF₂OCOC). |

¹⁹F-NMR spectrum in p.p.m. (with respect to CFCl₃=0) of isomer B:

| | |
|---|---|
| -50.0/-52.1 (2F, O-CF₂-O) ; | -70.0/-71.8 (2F, C-CF₂Cl) ; |
| -77.9 (1F, O-CFCl-C) ; | -54.6/-54.9 (3F, CF₃OC) : |
| -85.7/-86.1 (3F, OC(CF₃)O) ; | -100.3/-101.0 (1F, OCF(C)O). |

Mass spectrum (electronic impact) main peaks and respective intensities:
Product A: 69 (50); 119 (100); 151 (50); 185 (42); 251 (38);
Product B: 69 (96); 97 (50); 135 (42); 151 (92); 185 (100).

IR spectrum (cm⁻¹) , intensity of the mixture A 79%, B 21%:
(w)=weak, (m)=medium, (s)=strong, (vs)=very strong:
1388 (w); 1288 (vs); 1233 (vs); 1151 (vs); 1104 (vs); 1032 (s) ; 846 (m); 685 (w).

### EXAMPLE 3

### Synthesis of CF₃OCF₂CF₂OCF₂OCHClCHFCl perfluoro-1,2-dichloro-1,2-dihydro-3,5,8-trioxanonane (isomer C) and CF₃ OCF (CF₃) OCF₂OCHClCHFCl perfluoro-1,2-dichloro-1,2-dihydro-3,5,7-trioxa-6-methyloctane (isomer D).

In a reactor identical to that used in Example 1, maintained at the temperature of -112°C, 1.55 1/h of CF₂ (OF)₂ and 4.5 1/h of He are introduced through one of the two inlet pipes; through the second inlet pipe 1.4 1/h of CF₂=CF-OCF₃ and 0.7 1/h of He for 5 hours.

The residence time of the transport gas in the reaction zone comprised between the reactor outlet and the end of the two feeding pipes is of about 3 sec.

On the reactor bottom the reaction products are brought to room temperature and the gaseous mixture flow, monitored by gaschromatography, is fed in a continuous way, under mechanical stirring, into a second reactor identical to the one used for the same step in Example 1. Inside, the temperature is of -70°C and there are 50 g of 1,2-dichloroethylene CClH=CClH and 50 g of CFCl₃.

At the end of the addition of the reacting gases into the second reactor, after distillation of the solvent at room pressure, the reaction raw material is distilled by a plate column at the reduced pressure of 100 mmHg. 43.5 g of the mixture of the desired products (isomer C 78%, isomer D 22%, determined by gaschromaography) are collected. The molar yield of C+D with respect to the used CF₂(OF)₂ is 33%. The isomers have been separated by preparative gaschromatography.

### Characterization of products C and D

Mixture boiling point (C 78%, D 22%) at the reduced pressure of 100 mmHg: 71°C.

¹⁹F-NMR spectrum in p.p.m. (with respect to CFCl₃ = 0) of the isomer C perfluoro-1,2-dichloro-1,2-dihydro-3,5,8-trioxanonane:

| | |
|---|---|
| -56.0/-57.2 (2F, O-CF₂-O) ; | -143.2/-146.0 (1F, C-CHFCl) ; |
| -55.8 (3F, CF₃-OC) ; | -91.0/-91.4 (2F, C-OCF₂-C) ; |
| -90.3/-90.5 (2F, C-OC-CF₂OCOC). | |

¹⁹F-NMR spectrum in p.p.m. (with respect to CFCl₃=0) of the isomer D perfluoro-1,2-dichloro-1,2-dihydro-3,5,7-trioxa-6-methyloctane:

| | |
|---|---|
| -56.0/-57.2 (2F, O-CF₂-O) ; | -143.2/-146.0 (1F, C-CHFCl) ; |
| -54.9/-55.1 (3F, CF₃-OC) ; | -86.2/-86.3 (3F, OC(CF₃O) ; |
| -100.5/-101.0 (1F, OCF(C)O). | |

¹H spectrum in p.p.m. (with respect to TMS) of the isomers C and D: 6.28/6.05 (1H -CHFCl); 6.02/5.95 (1H -CHCl-)

Mass spectrum (electronic impact), main peaks and respective intensities %:
69 (84); 119 (100); 185 (51.1); 251 (84); 281 (15.8); 283 (4.8); 347 (5.7); 349 (1.7).

IR spectrum (cm⁻¹) intensity : (w)=weak, (m) =medium, (s)=strong, (vs)=very strong:
3001.0 (w) ; 2920.9 (w) ; 2850.9 (w) ; 1286.3 (vs) ; 1233.7 (vs) ; 1125.5 (vs) ; 1081.8 (s) ; 1047.9 (s) ; 815.9 (m) ; 766.3 (m).

### EXAMPLE 4

### Dehalogenation of perfluoro 1,2-dichloro-3,5-dioxaheptane

In a 25 ml three-necked flask, equipped with mechanical stirrer, thermometer, dropping funnel, distillation column equipped with water refrigerant and collecting trap maintained at -78°C and connected to a mechanical vacuum pump, 150 ml of DMF, 15 g of Zn in powder, 0.5 g of K₂CO₃ and 100 mg of I₂ are introduced. The internal temperature is brought to 80°C and 50 g of perfluoro -1,2-dichloro-3,5-dioxaheptane are added drop by drop. When the addition is over the mixture is allowed to react for about 30 minutes. At the end the internal pressure gradually brought from starting 760 mmHg to 300 mmHg. After about 20 minutes the collecting trap containing 34.2 g of perfluoro-3,5-dioxa-1-heptene (MOVE 1) is disconnected.

The dehalogenation yield is 85%.

### Characterization of perfluoro-3,5-dioxa-1-heptene (MOVE 1)

Boiling point at atmospheric pressure: 41.9°C.
¹⁹F-NMR spectrum in p.p.m. with respect to CFCl₃=0 :

| | |
|---|---|
| -56.8 (2F, O-CF₂-O) ; | -87.2 (3F, CF₃-C) ; |
| -90.6 (2F, C-CF₂-O) ; | -114 (1F, O-C=C-F) ; |
| -121.8 (1F, O-C=CF) ; | -137 (1F, O-C-F=C) ; |

Mass spectrum (electronic impact), main peaks and respective intensities:
69 (66.5%) ; 119 (100%) ; 147 (83.4%); 185 (89.4%); 216 (67.3%) ; 282 (8.2%).

IR spectrum (cm⁻¹) intensity: (w) =weak, (m) =medium, (s)=strong, (vs)=very strong:
1839.5 (m); 1407.6 (w); 1307.4 (vs); 1245.8 (vs); 1117.4 (vs); 907.2 (m); 846.0 (m).

### EXAMPLE 5

### Dehalogenation of the isomer mixture A+B obtained in Example 2 (perfluoro-1,2-dichloro-3,5,8-trioxanonane CF₃OCF₂CF₂CF₂OCFClCF₂Cl + perfluoro-1,2-dichloro-3,5,7-trioxa-6-methyloctane CF₃OCF(CF₃)OCF₂OCFClCF₂Cl).

In a 250 ml flask equipped as described in the previous Example 4, 110 ml of DMF, 10 g of Zn in powder and 0.3 ml of Br₂ are introduced. The internal temperature is brought to 75°C and 30.3 g of the binary mixture A+B separated in the previous Example 2 are added drop by drop. When the addition is over the mixture is allowed to ract for about 3 hours. At the end the internal pressure is gradually lowered from 760 mmHg to 200 mmHg at -79°C. After about 30 minutes the collecting trap is disconnected. The corresponding content, which is washed with water, is recovered. At the end 24.0 g of a mixture formed for 79% (gaschromatographic determination) by perfluoro-3,5,8-trioxa-1-nonene (MOVE 2) CF₃OCF₂CF₂OCF₂OCF=CF₂ (isomer A') and for 21% by perfluoro-3,5,7-trioxa-6,methyl-1 octene (MOVE 2a) CF₃OCF (CF₃) OCF₂O-CF=CF₂ (isomer B') are obtained, which are then separated by preparative gaschromatography.

### Characterization of products A' and B'

Boiling range of the isomer mixture at atmospheric pressure: 72.5°-74.5°C.

¹⁹F-NMR spectrum in p.p.m. (with respect to CFCl₃=0) of the isomer A':
-55.9 (3F, CF₃-O) ; -56.9 (2F, O-CF₂-O) ; -90.8 (2F, C-CF₂-O) ; -91.2 (2F, O-CF₂-C) ; -114 (1F, O-C=C-F) ; -121.8 (1F, -O-C=CF) ; -137 (1F, O-CF=C)
¹⁹F-NMR spectrum in p.p.m. (with respect to CFCl₃=0) of the isomer B':
-55.9 (3F, CF₃-O) ; -56.2 (2F, O-CF₂-O) ; -86.4 (3F, CF₃-C) ; -100.9 (1F, CF; -114 (1F, O-C=C-F); -122 (1F, O-C=CF); -137 (1F, O-CF=C).

Mass spectrum (electronic impact), main peaks and respective intensities of the isomer A':
69 (74); 81 (18); 119 (100); 147 (59); 185 (26); 251 (21);
Mass spectrum (electronic impact), main peaks and respective intensities of the isomer B':
69 (80); 81 (37); 97 (47); 119 (36); 147 (100); 185 (19).
IR spectrum (cm⁻¹intensity: (w) =weak, (m) =medium, (s) =strong, (vs) =very strong, 1839 (m); 1343 (s); 1248 (vs); 1145 (vs); 918 (m); 889 (m).

### EXAMPLE 6

### Dehalogenation of the isomers C+D mixture obtained in Example 3 (CF₃OCF₂CF₂OCF₂OCHClCHFCl perfluoro-1,2-dichloro-1,2-dihydro-3,5,8-trioxanonane (isomer C)+ CF₃OCF(CF₃)OCF₂OCHClCHFCl perfluoro-1,2-dichloro-1,2-dihydro-3,5,7-trioxa-6-methyloctane (isomer D)).

In a 500 ml three-necked flask, equipped with mechanical stirrer, thermometer, dropping funnel, distillation column having a water refrigerant and a collecting trap maintained at the temperature of -78°C, 250 ml of DMF, 30 g of zinc in powder and 300 mg of I₂ are introduced.

The temperature is brought to 100°C and 56.9 g of the isomer mixture obtained in Example 3 are added drop by drop.

When the addition is over the reactor internal temperature is brought to 120°C and stirring is maintained for 24 hours. At the end the reaction product, which contains traces of solvent and which is collected in the trap maintained at -78°C, is distilled. After washing with water 35 g of a mixture of perfluoro-1,2-dihydro-3-5-8-trioxa-1-nonene (isomer C', 79% by moles) and of perfluoro-1, 2-dihydro-3-5-7-trioxa-5-methyl-1-octene (isomer D', 21% by moles) are recovered. The isomers are separated by preparative gaschromatography.

The dehaloagenation reaction yield is 76%.

### Characterization of products C' and D'

Boiling range of the mixture of isomers C' 79%, D' 21% at atmospheric pressure: 90.0°-92.0°C.

¹⁹F -NMR spectrum in p.p.m. (with respect to CFCl₃=0) of the isomer C' perfluoro-1,2-dihydro-3,5,8-trioxa-1-nonene:

| | |
|---|---|
| -55.7 (3F, CF₃-O) ; | -57.3 (2F, O-CF₂-O) ; |
| -90.9 (2F, C-CF₂-O) ; | -91.2 (2F, O-CF₂-C) ; |
| -149.3/-150.0 (1F, O-C=C-F). | |

¹⁹F-NMR spectrum in p.p.m. (with respect to CFCl₃=0) of the isomer D' perfluoro-1,2-dihydro-3,5,7-trioxa-6-methyl-1-octene:

| | |
|---|---|
| -55.0 (3F, CF₃-O) ; | -56.9 (2F, O-CF₂-O) ; |
| -86.2 (3F, CF₃-C) ; | -101.0 (1F, CF). |
| -149.3/-150,0 (1F, O-C=C-F) | |

Mass spectrum (electronic impact), main peaks and respective intensities %:
69 (82); 119 (100); 185 (29); 246 (25); 251 (20); 312 (43).
IR spectrum (cm⁻¹) intensity of the isomer mixture (C' 79%, D' 21%): (w)=weak, (m)=medium, (s)=strong, (vs)=very strong:
3140 (w); 1722 (w); 1695 (w); 1402 (m); 1281 (vs); 1237 (vs); 1147 (vs); 1106 (vs); 1030 (m).

### EXAMPLE 7

### Crystalline copolymer between MOVE 1 and TFE.

A 5 l steel AISI 316 autoclave with stirrer working at 650 rpm has been used. After the vacuum has been effected, 3 l of demineralized water, 15.70 g of MOVE 1 and the microemulsion prepared according to the procedure described in USP 4,864,006 are in sequence introduced, so as to have a concentration of 2 g of surfactant/l of water.

The autoclave is heated up to 75°C and then 0.32 bar of ethane are introduced. A gaseous mixture in a molar ratio of 54.55 TFE/MOVE 1 is pumped by a compressor until having inside the autoclave a pressure of 21 absolute bar.

The composition of the gaseous mixture present in the autoclave top is analyzed by gaschromatography.

Before the reaction starting the gaseous phase results to be formed by the following molar percentages of the reactants: 93.1% TFE, 5.5% MOVE 1 and 1.4% Ethane. The reaction is triggered by feeding in a continuous way by a metering pump a potassium persulphate 0.0031 molar solution at a flow rate of 88 ml/h.

The pressure is maintained constant by feeding the monomer mixture. The polymer synthesis is stopped after 742 g of mixture have been fed in total.

The reactor is cooled at room temperature, the emulsion is discharged and the coagulation is induced by HNO₃ (65%) addition.

The polymer is separated, washed with water and dried at 220°C.

The IR analysis shows the presence of very small absorption bands in the carboxyl zone, whose intensity results to be half of the one obtained from a TFE/PVE copolymer film having the same thickness, prepared according to the comparative Example 3. MFI according to ASTMD 1238-52T was 4.4. The polymer therefore results thermally more stable (see the comparative Example hereunder).

### EXAMPLE 1 (comparative)

### Crystalline copolymer PVE/TFE (PFA).

One operates as in Example 7 except that in this case the perfluoropropylvinylether (PVE) is used instead of α-PDE to obtain a copolymer having MFI equal to that of the Examples 9.

The IR analysis shows absorption bands in the carboxyl zone, whose intensity is the double of that obtained from a TFE/MOVE 1 copolymer film having an equal thickness prepared according to Example 9.

## Claims

1. Fluorovinylethers having the following formula:
CFX=CXOCF₂OR (I)
wherein R is a C₂-C₆ linear, branched or C₅-C₆ cyclic (per)fluoroalkyl group, or a C₂-C₆ linear, branched (per)fluoro oxyalkyl group containing from one to three oxygen atoms; when R is a fluoroalkyl or fluorooxyalkyl group as above defined it can contain from 1 to 2 atoms, equal to or different from each other, selected from the following: H, Cl, Br, I; X = H.

2. Fluorovinylethers according to claim 1, having the formula:
CFX=CXOCF₂OCF₂CF₂Y (II)
wherein Y = F, OCF₃; X as above defined.

3. Homopolymers and polymers obtainable by polymerizing the fluorovinylethers having formula
CFX=CXOCF₂OR (I)
wherein R is a C₂-C₆ linear, branched or C₅-C₆ cyclic (per)fluoroalkyl group, or a C₂-C₆ linear, branched (per)fluoro oxyalkyl group containing from one to three oxygen atoms; when R is a fluoroalkyl or fluorooxyalkyl group as above defined it can contain from 1 to 2 atoms, equal to or different from each other, selected from the following: H, Cl, Br, I; X = F, H;
with at least another polymerizable monomer, wherein the (per)fluoro polymers are plastomeric ones since they contain an amount of vinylether of claims 1-4 which lead to the formation of crystalline zones.

4. Polymers according to claim 3 wherein the fluorovinylethers have formula:
CFX=CXOCF₂OCF₂CF₂Y (II)
wherein Y = F, OCF₃; X as defined in claim 3

5. Polymers according to claims 3-4 wherein the fluorovinylethers have formula:
CF₂=CFOCF₂OCF₂CF₂Y (III)
wherein Y is as above defined.

6. Polymers according to claims 3-5 wherein the fluorovinylether has formula:
CF₂=CFOCF₂OCF₂CF₃ (IV)

7. Copolymers according to claim 3-6, wherein the comonomers are fluorinated compounds having at least one polymerizable double bond C=C, optionally containing hydrogen and/or chlorine and/or bromine and/or iodine and/or oxygen.

8. Copolymers according to claims 3-7, wherein optional copolymerizable comonomers are non fluorinated olefins C₂-C₈.

9. Copolymers according to claims 3-8, wherein the comonomers are selected from the following:
- C₂-C₈ perfluoroolefins, preferably tetrafluoroethylene (TFE), hexafluoropropene (HFP), hexafluoroisobutene;
- C₂-C₈ hydrogenated fluoroolefins, preferably vinyl fluoride (VF), vinylidene fluoride (VDF), trifluoroethylene, CH₂=CH-R²_{f} perfluoroalkylethylenes, wherein R²_{f} is a C₁-C₆ perfluoroalkyl;
- C₂-C₈ chloro- and/or bromo- and/or iodo-fluoroolefins, preferably chlorotrifluoroethylene (CTFE) and bromotrifluoroethylene;
- CF₂=CFOR²_{f} (per)fluoroalkylvinylethers (PAVE), wherein R²_{f} is a C₁-C₆ (per)fluoroalkyl, preferably trifluoromethyl, bromodifluoromethyl or heptafluoropropyl;
- CF₂=CFOX^{a} (per) fluoro-oxyalkylvinylethers, wherein X^{a} is: a C₁-C₁₂ alkyl, or a C₁-C₁₂ oxyalkyl, or a C₁-C₁₂ (per) fluorooxyalkyl having one or more ether groups, preferably perfluoro-2-propoxy-propyl;
- sulphonic monomers having the structure CF₂=CFOX^{b}SO₂F, wherein X^{b} = CF₂CF₂, CF₂CF₂CF₂, CF₂CF (CF₂X^{c}) wherein X^{c} = F, Cl, Br.

10. A synthesis process of the (per) fluorovinylethers of claims 1-2 comprising the initial reaction of hypofluorite with a fluorinated olefin having the formula R₁R₂C=CR₃R₄ to give the intermediate hypofluorite F-CR₁R₂-CR₃R₄-OCF,OF, the subsequent reaction of this compound with a second fluorinated olefin of formula R₅R₆C=CR₇R₈ to give the intermediate F-CR₁R₂-CR₃R₄-OCF₂O-CR₅R₆-CR₇R₈-F, which by subsequent dehalogenation or dehydrohalogenation leads to the obtainment of the new perfluorovinylethers, according to the scheme:
a)
CF₂(OF)₂ + R₁R₂C=CR₃R₄ →F-CR₁R₂-CR₃R₄-OCF₂OF (VI)
b)
F-CR₁R₂-CR₃R₄-OCF₂OF + R₅R₆C²=C¹R₇R₈ -----→ F-CR₁R₂-CR₃R₄-OCF₂O-C²R₅R₆-C¹R₇R₈-F (VII)
c) 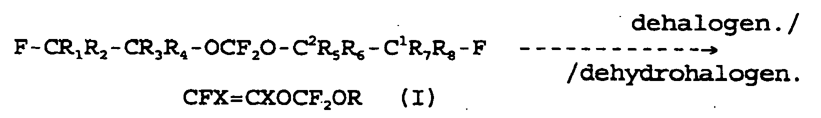
wherein:
- in the compound (VII):
- R₁, R₄, equal or different, are H, F; R₂, R₃, equal or different are H, Cl under the following conditions: (1) when the final reaction is a dehalogenation R₂, R₃ = Cl, (2) when the final reaction is a dehydrohalogenation one of the two substituents R₂, R₃ is H and the other is Cl;
- R₅, R₆, R₇, R₈ are :
- F, or one of them is a C₁-C₄ linear or branched perfluoroalkyl group, or a C₁-C₄ linear or branched perfluorooxyalkyl group containing from one to three oxygen atoms, or R₅ and R₇, or R₆ and R₈, are linked each other to form with C² and C¹ a C₅-C₆ cycle perfluoroalkyl group;
- when one of the R₅ to R₈ radicals is a C₂-C₄ linear or branched fluoroalkyl, or a C₂-C₄ linear or branched fluorooxyalkyl group containing from one to three oxygen atoms, one or two of the other R₅ to R₈ are F and one or two of the remainders, equal to or different from each other, are selected from H, Cl, Br, Iodine; when the substituents selected from H, Cl, Br, Iodine are two, they are both linked to the same carbon atom; when R₅ and R₇, or R₆ and R₈, are linked each other to form with C² and C¹ a C₅-C₆ cycle fluoroalkyl group, one of the two free substituents R₆, R₈ or R₅, R₇ is F and the other is selected from H, Cl, Br, Iodine.
- the fluoroalkene used in the reaction a) is replaceable with that of the subsequent reaction b); in this case the meanings defined for the substituents of the R₁-R₄ group, and respectively of the R₅-R₈ group, are interchangeable each other, with the proviso that the position of each radical of each of the two groups R₁-R₄ and R₅-R₈ with respect to
- OCF₂O- on the chain of the intermediate compound (VII), is the same which is occupied if the synthesis takes place according to the above reported scheme and the two olefins each react in the considered steps.

11. A process according to claim 12, wherein in step a) the reactants are in an approximately unitary molar ratio, or with an excess of CF₂(OF)₂, the residence time of the mixture in the reactor ranges from few hundredths of second up to about 120 seconds, the reaction temperature from -40 to -150°C and the compound (VI) is not separated and is transferred in a continuous way into the reaction of step b).

12. A process according to claims 12 and 13, wherein in the step b) the R₅R₆C=CR₇R₈ olefin at a pure state or in solution, reacts with the product obtained in the first reaction with formation of the compound (VII) ; the olefin is fed in a continuous way, so as to maintain its concentration constant in the reactor; the temperature of the reaction b) can range from -50° to - 130°C, preferably from -20° to -100°C, the olefin concentration is higher than or equal to 0.01M, preferably the concentration is higher than 3M, more preferably also the pure compound can be used.

13. A process according to claims 12 and 14, wherein in the step c) the compound (VII), dependently on the olefins used in steps a) and b), after distillation from the reaction raw material, is subjected to dechlorination-or to dehydrochlorination to obtain the vinylethers of formula (I).

14. A synthesis process of the (per)fluorovinylethers according to claims 12-15, wherein the compound (VII) is obtainable by reaction of a hypofluorite of formula X₁X₂C (OF)₂ wherein X₁ and X₂ equal or different are F, CF₃, and two fluoroalkenes of formula respectively R^{A}₁R^{A}₂C=CR^{A}₃R^{A}₄ and R^{A}₅R^{A}₆C=CR^{A}₇R^{A}₈, wherein R^{A}₁**-**R^{A}₈ equal or different, are F, H, Cl, Br, I, -CF₂OSO₂F, -SO₂F, -COF, C₁-C₅ linear or branched perfluoroalkyl or oxyperfluoroalkyl group.

## Patentansprüche

1. Fluorvinylether mit der folgenden Formel :
CFX=CXOCF₂OR (I)
worin R für eine lineare oder verzweigte C₂-C₆-(Per)fluoralkylgruppe oder eine cyclische C₅-C₆-(Per)fluoralkylgruppe oder eine lineare oder verzweigte C₂-C₆-(Per)fluoroxyalkylgruppe mit einem bis drei Sauerstoffatomen steht; wenn R für Fluoralkyl oder Fluoroxyalkyl gemäß obiger Definition steht, kann es 1 bis 2 Atome, die gleich oder verschieden sind und unter H, Cl, Br, I ausgewählt sind, enthalten; X = H.

2. Fluorvinylether nach Anspruch 1 mit der Formel :
CFX=CXOCF₂OCF₂CF₂Y (II)
worin Y = F, OCF₃ und X die oben angegebene Bedeutung besitzt.

3. Homopolymere und Polymere, die durch Polymerisation der Fluorvinylether mit der Formel
CFX=CXOCF₂OR (I)
worin R für eine lineare oder verzweigte C₂-C₆-(Per)fluoralkylgruppe oder eine cyclische C₅-C₆-(Per)fluoralkylgruppe oder eine lineare oder verzweigte C₂-C₆-(Per)fluoroxyalkylgruppe mit einem bis drei Sauerstoffatomen steht; wenn R für Fluoralkyl oder Fluoroxyalkyl gemäß obiger Definition steht, kann es 1 bis 2 Atome, die gleich oder verschieden sind und unter H, Cl, Br, I ausgewählt sind, enthalten; X = F, H;
mit mindestens einem anderen polymerisierbaren Monomer erhältlich sind,
wobei die (Per)fluorpolymere plastomer sind, da sie eine Menge an Vinylether gemäß den Ansprüchen 1-4 enthalten, die zur Bildung von kristallinen Zonen führt.

4. Polymere nach Anspruch 3, worin die Fluorvinylether die Formel :
CFX=CXOCF₂OCF₂CF₂Y (II)
worin Y = F, OCF₃ und X die in Anspruch 3 angegebene Bedeutung besitzt, aufweisen.

5. Polymere nach den Ansprüchen 3-4, worin die Fluorvinylether die Formel :
CF₂=CFOCF₂OCF₂CF₂Y (III)
worin Y die oben angegebene Bedeutung besitzt, aufweisen.

6. Polymere nach den Ansprüchen 3-5, worin die Fluorvinylether die Formel :
CF₂=CFOCF₂OCF₂CF₃ (IV)
aufweisen.

7. Copolymere nach den Ansprüchen 3-6, worin es sich bei den Comonomeren um fluorierte Verbindungen mit mindestens einer polymerisierbaren C=C-Doppelbindung, die gegebenenfalls Wasserstoff und/oder Chlor und/oder Brom und/oder Iod und/oder Sauerstoff enthalten, handelt.

8. Copolymere nach den Ansprüchen 3-7, worin es sich bei fakultativen copolymerisierbaren Comonomeren um nichtfluorierte C₂-C₈-Olefine handelt.

9. Copolymere nach den Ansprüchen 3-8, worin die Comonomere unter den folgenden ausgewählt sind :
- C₂-C₈-Perfluorolefinen, vorzugsweise Tetrafluorethylen (TFE), Hexafluorpropen (HFP) und Hexafluorisobuten;
- hydrierten C₂-C₈-Fluorolefinen, vorzugsweise Vinylfluorid (VF), Vinylidenfluorid (VDF), Trifluorethylen, Perfluoralkylethylenen CH₂=CH-R²_{f}, worin R²_{f} für C₁-C₆-Perfluoralkyl steht;
- C₂-C₈-Chlor- und/oder -Brom- und/oder Iodfluor-olefinen, vorzugsweise Chlortrifluorethylen (CTFE) und Bromtrifluorethylen;
- (Per)fluoralkylvinylethern (PAVE) CF₂=CFOR²_{f}, worin R²_{f} für C₁-C₆-Perfluoralkyl, vorzugsweise Trifluormethyl, Bromdifluormethyl oder Heptafluorpropyl, steht;
- (Per)fluoroxyalkylvinylethem CF₂=CFOX^{a}, worin X^{a} für C₁-C₁₂-Alkyl, C₁-C₁₂-Oxyalkyl oder C₁-C₁₂-(Per)fluoroxyalkyl mit einer oder mehreren Ethergruppen, vorzugsweise Perfluor-2-propoxypropyl, steht;
- Sulfonmonomeren der Struktur CF₂=CFOX^{b}SO₂F, worin X^{b} = CF₂CF₂, CF₂CF₂CF₂, CF₂CF(CF₂X^{c}), worin X^{c} = F, Cl, Br.

10. Verfahren zur Synthese von (Per)fluorvinylethern gemäß den Ansprüchen 1-2, bei dem man zunächst Hypofluorit mit einem fluorierten Olefin mit der Formel R₁R₂C=CR₃R₄ zum Hypofluorit-Zwischenprodukt F-CR₁R₂-CR₃R₄-OCF₂OF umsetzt und diese Verbindung danach mit einem zweiten fluorirten Olefin der Formel R₅R₆C=CR₇R₈ zum Zwischenprodukt F-CR₁R₂-CR₃R₄-OCF₂O-CR₅R₆-CR₇R₈-F umsetzt, welches durch nachfolgende Dehalogenierung oder Dehydrohalogenierung zum Erhalt der neuen Perfluorvinylether gemäß dem folgenden Schema führt :
a)
CF₂(OF)₂ + R₁R₂C=CR₃R₄ → F-CR₁R₂-CR₃R₄-OCF₂OF (VI)
b)
F-CR₁R₂-CR₃R₄-OCF₂OF + R₅R₆C²=C¹R₇R₈ → F-CR₁R₂-CR₃R₄-OCF₂O-C²R₅R₆-C¹R₇R₈-F (VII)
c) 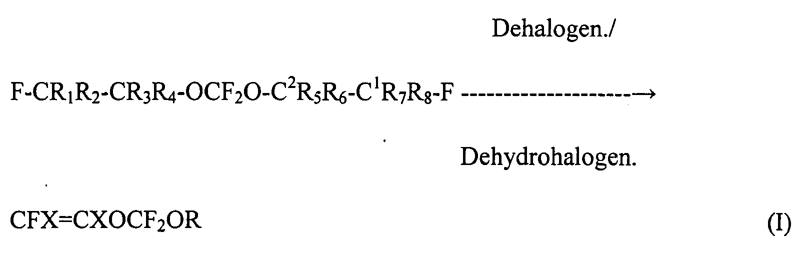
worin :
- in der Verbindung (VII) :
- R₁ und R₄ gleich oder verschieden sind und für H oder F stehen; R₂ und R₃ gleich oder verschieden sind und für H oder Cl stehen, unter den folgenden Bedingungen : (1) wenn es sich bei der letzten Reaktion um eine Dehalogenierung handelt, R₂, R₃ = Cl; (2) wenn es sich bei der letzten Reaktion um eine Dehydrohalogenierung handelt, steht einer der beiden Substituenten R₂ und R₃ für H und der andere für Cl;
- R₅, R₆, R₇ und R₈ für
- F stehen oder einer davon für eine lineare oder verzweigte C₁-C₄-Perfluoralkylgruppe oder eine lineare oder verzweigte C₁-C₄-Perfluoroxyalkylgruppe mit einem bis drei Sauerstoffatomen steht, oder R₅ und R₇ oder R₆ und R₈ miteinander verbunden sind und mit C² und C¹ eine cyclische C₅-C₆-Perfluoralkylgruppe bilden;
- wenn einer der Reste R₅ bis R₈ für eine lineare oder verzweigte C₂-C₄-Fluoralkylgruppe oder eine lineare oder verzweigte C₂-C₄-Fluoroxyalkylgruppe mit einem bis drei Sauerstoffatomen steht, einer oder zwei der anderen Reste R₅ bis R₈ für F stehen und einer oder zwei der übrigen Reste gleich oder voneinander verschieden sind und unter H, Cl, Br und Iod ausgewählt sind; wenn zwei unter H, Cl, Br und Iod ausgewählte Substituenten vorhanden sind, diese beide an das gleiche Kohlenstoffatom gebunden sind; wenn R₅ und R₇ oder R₆ und R₈ miteinander verbunden sind und mit C² und C¹ eine cyclische C₅-C₆-Perfluoralkylgruppe bilden, einer der beiden freien Substituenten R₆, R₈ bzw. R₅, R₇ für F steht und der andere unter H, Cl, Br und Iod ausgewählt ist;
- das bei der Reaktion a) verwendete Fluoralken durch dasjenige der nachfolgenden Reaktion b) ersetzt werden kann; in diesem Fall sind die für die Substituenten der Gruppe R₁-R₄ definierten Bedeutungen und respektive der Gruppe R₅-R₈ untereinander austauschbar, mit der Maßgabe, daß die Position jedes Rests jeder der beiden Gruppen R₁-R₄ und R₅-R₈ bezüglich -OCF₂O- in der Kette der Zwischenverbindung (VII) die gleiche ist, die besetzt wird, wenn die Synthese gemäß dem oben angegebenen Schema erfolgt und die beiden Olefine jeweils in den betrachteten Schritten reagieren.

11. Verfahren nach Anspruch 12, bei dem in Schritt a) die Reaktanten in ungefähr unitärem Molverhältnis oder mit einem Überschuß an CF₂(OF)₂ vorliegen, die Verweilzeit der Mischung im Reaktor im Bereich von einigen Hundertstel einer Sekunde bis etwa 120 Sekunden beträgt, die Reaktionstemperatur -40 bis -150°C beträgt und die Verbindung (VI) nicht abgetrennt und kontinuierlich in die Reaktion von Schritt b) überführt wird.

12. Verfahren nach den Ansprüchen 12 und 13, bei dem in Schritt b) das Olefin R₅R₆C=CR₇R₈ in reinem Zustand oder in Lösung mit dem bei der ersten Reaktion erhaltenen Produkt unter Bildung der Verbindung (VII) reagiert; das Olefin kontinuierlich zugeführt wird, um seine Konzentration im Reaktor konstant zu halten; die Temperatur der Reaktion b) im Bereich von -50 bis -130°C, vorzugsweise -20 bis -100°C, liegen kann, die Olefinkonzentration größer gleich 0,01 M und vorzugsweise größer 3 M ist und besonders bevorzugt auch die reine Verbindung verwendet werden kann.

13. Verfahren nach den Ansprüchen 12 und 14, bei dem man in Schritt c) die Verbindung (VII) je nach den in den Schritten a) und b) verwendeten Olefinen nach Destillation aus dem Reaktionsrohmaterial einer Dechlorierung oder Dehydrochlorierung unterwirft, wobei man die Vinylether der Formel (I) erhält.

14. Verfahren zur Synthese der (Per)fluorvinylether nach den Ansprüchen 12-15, bei dem die Verbindung (VII) durch Umsetzung eines Hypofluorits der Formel X₁X₂C(OF)₂, worin X₁ und X₂ gleich oder verschieden sind und für F oder CF₃ stehen, und zwei Fluoralkenen der Formel R^{A}₁R^{A}₂C=CR^{A}₃R^{A}₄ bzw. R^{A}₅R^{A}₆C=CR^{A}₇R^{A}₈, worin R^{A}₁-R^{A}₈ gleich oder verschieden sind und für F, H, Cl, Br, I, -CF₂OSO₂F, -SO₂F, -COF oder eine lineare oder verzweigte C₁-C₅-Perfluoralkyl- oder Oxyperfluoralkylgruppe stehen, erhältlich ist.

## Revendications

1. Ethers de fluorovinyle ayant la formule :
CFX=CXOCF₂OR (I)
dans laquelle R est un groupe (per)fluoroalkyle linéaire en C₂-C₆, ramifié ou cyclique en C₅-C₆, ou un groupe (per)fluorooxyalkyle linéaire en C₂-C₆, ramifié contenant de 1 à 3 atome(s) d'oxygène ; lorsque R est un groupe fluoroalkyle ou fluorooxyalkyle tel que défini ci-dessus il peut contenir de 1 à 2 atome(s), égaux ou différents l'un de l'autre, choisis parmi les suivants : H, Cl, Br, I ; X = H.

2. Ethers de fluorovinyle selon la revendication 1, ayant la formule :
CFX=CXOCF₂OCF₂CF₂Y (II)
dans laquelle Y = F, OCF₃ ; X est tel que défini ci-dessus.

3. Homopolymères et polymères pouvant être obtenus par polymérisation des éthers de fluorovinyle de formule
CFX=CXOCF₂OR (I)
dans laquelle R est un groupe (per)fluoroalkyle linéaire en C₂-C₆, ramifié ou cyclique en C₅-C₆, ou un groupe (per)fluorooxyalkyle linéaire en C₂-C₆, ramifié contenant de un à trois atome(s) d'oxygène ; lorsque R est un groupe fluoroalkyle ou fluorooxyalkyle tel que défini ci-dessus il peut contenir de 1 à 2 atome(s), égaux ou différents l'un de l'autre, choisis parmi les suivants : H ; Cl, Br, I; X = F, H;
avec au moins un autre monomère polymérisable, dans lesquels les (per)fluoropolymères sont des plastomères dans la mesure où ils contiennent une quantité d'éther de vinyle selon les revendications 1 à 4 qui conduisent à la formation de zones cristallines.

4. Polymères selon la revendication 3, dans lesquels les éthers de fluorovinyle sont de formule :
CFX=CXOCF₂OCF₂CF₂Y (II)
dans laquelle Y = F, OCF₃ ; X tel que défini selon la revendication 3.

5. Polymères selon les revendications 3 à 4, dans lesquels les éthers de fluorovinyle ont la formule :
CF₂=CFOCF₂OCF₂CF₂Y (III)
dans laquelle Y est tel que défini ci-dessus.

6. Polymères selon les revendications 3 à 5, dans lesquels l'éther de fluorovinyle a la formule :
CF₂=CFOCF₂OCF₂CF₃ (IV).

7. Copolymères selon les revendications 3 à 6, dans lesquels les comonomères sont des composés fluorés ayant au moins une double liaison polymérisable C=C, contenant facultativement de l'hydrogène et/ou du chlore et/ou du brome et/ou de l'iode et/ou de l'oxygène.

8. Copolymères selon les revendications 3 à 7, dans lesquels les comonomères copolymérisables facultatifs sont des oléfines non fluorées en C₂-C₈.

9. Copolymères selon les revendications 3 à 8, dans lesquels les comonomères sont choisis parmi les suivants :
- perfluorooléfines en C₂-C₈, de préférence tétrafluoroéthylène (TFE), hexafluoropropène (HFP), hexafluoroisobutène ;
- fluorooléfines hydrogénées en C₂-C₈, de préférence fluorure de vinyle (VF), fluorure de vinylidène (VDF), trifluoroéthylène, CH₂=CH-R²_{f} perfluoroalkyléthylènes, dans lesquels R²_{f} est un groupe perfluoroalkyle en C₁-C₆;
- chloro- et/ou bromo- et/ou iodo-fluorooléfines en C₂-C₈, de préférence chlorotrifluoroéthylène (CTFE) et bromotrifluoroéthylène ;
- CF₂=CFOR²_{f} éthers de (per)fluoroalkylvinyle (PAVE), dans lesquels R²_{f} est un groupe (per)fluoroalkyle en C₁-C₆, de préférence trifluorométhyle, bromodifluorométhyle ou heptafluoropropyle ;
- CF₂=CFOX^{a} éthers de (per)fluoro-oxyalkylvinyle, dans lesquels X^{a} est : un groupe alkyle en C₁-C₁₂, ou un groupe oxyalkyle en C₁-C₁₂, ou un groupe (per)fluorooxyalkyle en C₁-C₁₂ ayant un ou plusieurs groupe(s) éther, de préférence perfluoro-2-propoxy-propyle ;
- monomères sulfoniques ayant la structure CF₂=CFOX^{b}SO₂F, dans lesquels X^{b} = CF₂CF₂, CF₂CF₂CF₂, CF₂CF(CF₂X^{c}) dans lesquels X^{c} = F, CI, Br.

10. Procédé de synthèse des éthers de (per)fluorovinyle selon les revendications 1 à 2 comprenant la réaction initiale d'hypofluorite avec une oléfine fluorée ayant la formule R₁R₂C=CR₃R₄ pour donner l'hypofluorite intermédiaire F-CR₁R₂-CR₃R₄-OCF₂OF, la réaction ultérieure de ce composé avec une seconde oléfine fluorée de formule R₅R₆C=CR₇R₈ pour donner l'intermédiaire F-CR₁R₂-CR₃R₄-OCF₂O-CR₅R₆-CR₇R₈-F, qui par déshalogénation ultérieure ou déshydrohalogénation conduit à l'obtention des nouveaux éthers de perfluorovinyle, selon le schéma :
a)
CF₂(OF)₂ + R₁R₂C=CR₃R₄ → F-CR₁R₂-CR₃R₄-OCF₂OF (VI)
b)
F-CR₁R₂-CR₃R₄-OCF₂OF + R₅R₆C²=C¹R₇R₈ -----→ F-CR₁R₂-CR₃R₄-OCF₂ O-C²R₅R₆-C¹R₇R₈-F (VII)
c) 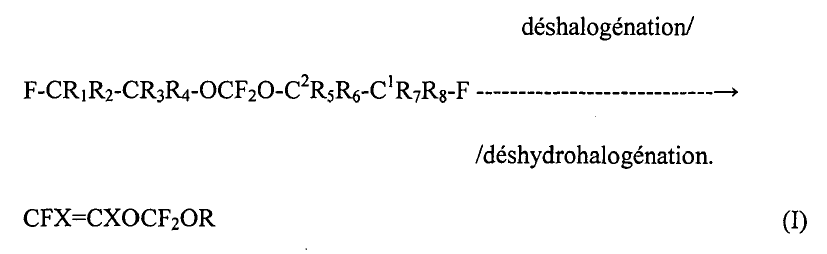
dans lesquelles :
- dans le composé (VII) :
- R₁, R₄, égaux ou différents, sont H, F ; R₂, R₃, égaux ou différents sont H, Cl sous les conditions suivantes : (1) lorsque la réaction finale est une déshalogénation R₂, R₃ = Cl, (2) lorsque la réaction finale est une déshydrohalogénation l'un des deux substituants R₂, R₃ est H et l'autre est Cl ;
- R₅, R₆, R₇, R₈ sont :
- F, ou l'un d'eux est un groupe perfluoroalkyle linéaire ou ramifié en C₁-C₄, ou un groupe perfluorooxyalkyle linéaire ou ramifié en C₁-C₄ contenant de un à trois atome(s) d'oxygène ou R₅ et R₇, ou R₆ et R₈, sont liés l'un à l'autre pour former avec C² et C¹ un groupe perfluoroalkyle cyclique en C₅-C₆ ;
- lorsque l'un des radicaux R₅ et R₈ est un groupe fluoroalkyle linéaire ou ramifié en C₂-C₄, ou un groupe fluorooxyalkyle linéaire ou ramifié en C₂-C₄ contenant de un à trois atome(s) d'oxygène, un ou deux de l'autre R₅ à R₈ sont F et l'un ou deux des restants, égaux ou différents l'un de l'autre sont choisis parmi H, Cl, Br, l'iode ; lorsque les substituants choisis parmi H, CI, Br, l'iode sont au nombre de deux, ils sont tous deux liés au même atome de carbone ; lorsque R₅ et R₇, ou R₆ et R₈, sont liés l'un à l'autre pour former avec C² et C¹ un groupe fluoroalkyle cyclique en C₅-C₆, l'un des deux substituants libres R₆, R₈ ou R₅, R₇ est F et l'autre est choisi parmi H, Cl, Br, l'iode.
- le fluoroalcène utilisé dans la réaction a) est remplaçable par celui de la réaction ultérieure b) ; dans ce cas les significations définies pour les substituants du groupe R₁-R₄, et respectivement du groupe R₅-R₈, sont interchangeables l'une par l'autre, à condition que la position de chaque radical de chacun des deux groupes R₁-R₄ et R₅-R₈ par rapport à -OCF₂O-sur la chaîne du composé intermédiaire (VII), soit le même qui est occupé si la synthèse a lieu selon le schéma rapporté ci-dessus et les deux oléfines réagissent chacune dans les étapes considérées.

11. Procédé selon les revendications 12, dans lequel dans l'étape a) les réactifs sont sous un rapport molaire approximativement unitaire, ou avec un excès de CF₂(OF)₂, le temps de résidence du mélange dans le réacteur s'étend de quelques centaines de secondes jusqu'à environ 120 secondes, la température réactionnelle de-40 à -150°C et le composé (VI) n'est pas séparé et est transféré d'une manière continue dans la réaction de l'étape b).

12. Procédé selon les revendications 12 et 13, dans lequel dans l'étape b) l'oléfine R₅R₆C=CR₇R₈ sous un état pur ou en solution, réagit avec le produit obtenu dans la première réaction avec la formation du composé (VII) ; l'oléfine est alimentée d'une manière continue, afin de maintenir sa concentration constante dans le réacteur ; la température de la réaction b) peut s'étendre de 50°C à -130°C, de préférence de-20°C à -100°C, la concentration de l'oléfine est supérieure ou égale à 0,01 M, de préférence la concentration est supérieure à 3 M, plus préférablement également le composé pur peuvent être utilisé.

13. Procédé selon les revendications 12 et 14, dans lequel dans l'étape c) le composé (VII), dépendamment des oléfines utilisées dans les étapes a) et b) après la distillation de la matière première réactionnelle, est soumis à la déchloration ou déshydrochloration pour obtenir les éthers de vinyle selon la formule (I).

14. Procédé de synthèse des éthers de (per)fluorovinyle selon les revendication 12 à 15, dans lequel le composé (VII) peut être obtenu par la réaction d'une hypofluorite de formule X₁X₂C(OF)₂ dans laquelle X₁ et X₂ égaux ou différents sont F, CF₃ et deux fluoroalcènes de formule respectivement R^{A}₁R^{A}₂C=CR^{A}₃R^{A}₄, et R^{A}₅R^{A}₆C=CR^{A}₇R^{A}₈, dans lesquelles R^{A}₁-R ^{A}₈ égaux ou différents, sont F, H, CI, Br, I, -CF₂OSO₂F, -SO₂F, -COF, un groupe perfluoroalkyle ou oxyperfluoroalkyle linéaire ou ramifié en C₁-C₅.
